Europäisches Patentamt

European Patent Office

Office européen des brevets

(19)

(11) **EP 1 411 370 A1**

(12) **EUROPEAN PATENT APPLICATION**

(43) Date of publication:
**21.04.2004 Bulletin 2004/17**

(51) Int Cl.⁷: **G01S 15/89**, G01S 7/52,
A61B 8/14

(21) Application number: **03023280.5**

(22) Date of filing: **15.10.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **15.10.2002 JP 2002300397**

(71) Applicant: **MATSUSHITA ELECTRIC INDUSTRIAL
CO., LTD.**
**Kadoma-shi, Osaka 571-8501 (JP)**

(72) Inventor: **Ohmiya, Jun**
**Katano-shi, 576-0021 (JP)**

(74) Representative: **Balsters, Robert et al**
**Novagraaf International SA**
**25, avenue du Pailly**
**1220 Les Avanchets - Geneva (CH)**

(54) **Image processing apparatus, method and program**

(57)     An image processing apparatus for sending ultrasound from an ultrasound probe and generating images based on reflection signals generated from echoes from a check object, comprising: a specifying unit operable to specify at least two frequency bands in reflection signals; a generating unit (104) operable to generate images for the respective frequency bands based on the signal having the specified frequency band in reflection signals; and a synthesizing unit (106) operable to synthesize images respectively for the generated frequency bands.

Fig. 12

**Description**

**BACKGROUND OF THE INVENTION**

**(1) Field of the Invention**

The present invention relates to an image processing apparatus such as an ultrasound diagnosis apparatus for non-destructively displaying inside the check object.

**(2) Description of the Related Art**

An image processing apparatus for irradiating a sound signal on a subject, receiving the signals reflected on the subject and generating an image of inside the subject based on these received signals is widely used in the industrial field or the medical field. One of representative examples of apparatuses like this is an ultrasound diagnosis apparatus.

These image processing apparatuses are becoming capable of measuring the subject and generating an image in real time because they are improved in processing speed thanks to the higher performance of the recent computers. Also, in the clinical division where image processing apparatuses with a high temporal resolution are required such as in the division of circulatory organs of the medical field, it becomes possible to provide a tomography image of a check object in real time and realize a diagnosis of a living organ using these images.

The ultrasound diagnosis apparatus sends a sound signal to the subject, receives the echoes, and generates images based on these echoes. Therefore, when there exists a part that does not allow a sound signal to pass through easily or when there exists a part (boundary) that reflects almost all the sound signal, a low luminance area called shadow may emerge because it is impossible to obtain the information on the inner part of the part (the area which is farer from the probe that sends and receives ultrasound).

Also, interference occurs because a sound signal is an undulation. When the distance between scatterers is shorter than the wavelength of the sound signal is not more than one wavelength of the sound signal, a strong randomness may appear because of this subject distribution. False information produced by these causes is called speckle noises (which is also called salt and pepper noises).

In order to eliminate the influence of a shadow or speckle noises, several image data that are obtained in a plurality of steering angle directions are synthesized into a single image. This method is called compound scan. Generally, a compound scan is performed by arithmetic averaging several image data that are obtained in a plurality of directions.

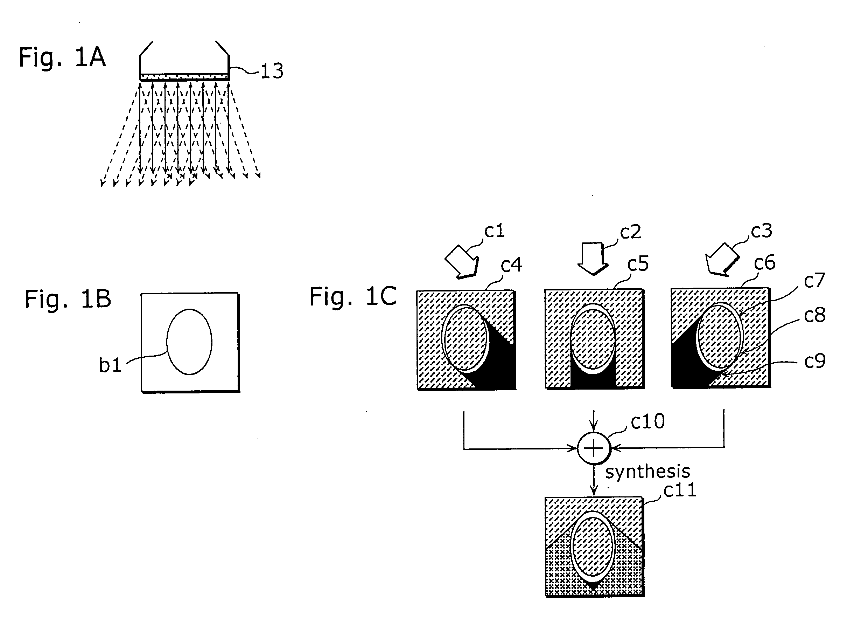

Fig. 1A ～ 1C are diagrams showing a method of general compound scan. Fig. 1A shows how an ultrasound beam is irradiated from an array ultrasound probe in a plurality of directions.

In the case of a check object b1 shown in Fig. 1B, it is possible to obtain image data c4, c5 and c6 shown in Fig. 1C when scanning the check object using an array ultrasound probe 13 as shown in Fig. 1C by an electric steering in a plurality of directions c1, c2 and c3 shown in Fig. 1C. The respective image data include ( i ) c7 where the edge of the check object emerges under high luminance, ( ii ) c8 where the edge of the check object emerges under low luminance because the reflection signal is very weak and ( iii ) c9 which is a shadow part where no sound signal is reachable or no reflection signal is detectable. The way these parts of ( i ) the high luminance part, ( ii ) the low luminance part and ( iii ) the shadow part emerge varies because the respective image data c4 ～ c6 are obtained in different steering angle directions.

By synthesizing those images that are obtainable in a plurality of directions, it becomes possible to generate a synthesized image with little missing information. An example of the synthesis result c11 shown in Fig. 1C shows that a conventional method is suffice to obtain enough effect in improving the (ii) and (iii). Generally, a compound scan is performed by arithmetic averaging several image data that are obtained in a plurality of directions. This method makes it possible to narrow a shadow part and also eliminate white noises.

[Patent literacy]
Japanese Laid-Open Patent application No. 09-094248

[Non-patent literacy]
Handbook of Ultrasound Apparatus for Medical Use (revised version) supervised by Electronic Industries Association of Japan Corp. published by Corona publishing Co., LTD. issued on January 20th, 1997.

However, there is a problem that it is impossible to eliminate speckle noises completely by compound scan.

As mentioned above, speckle noises are generated by interference between echoes when a distribution of a scatterer shorter than a wavelength exists in a check object. In other words, the appearing pattern of speckle noises is determined depending on ( i ) the positional relationship between an array ultrasound probe and a check object and ( ii ) the wavelength of a sound signal to be used. The appearing pattern of speckle noises changes because the way of interference between echoes changes by obtaining image data in different directions, but there is no big change in appearing pattern of speckle noises because the difference in angle between the respective image data is only 15° when obtaining data in the three directions as shown in Fig. 1C because the maximum difference in angle can be made by an array ultrasound probe is generally about 30° . In this case, speckle noises are not sufficiently eliminated.

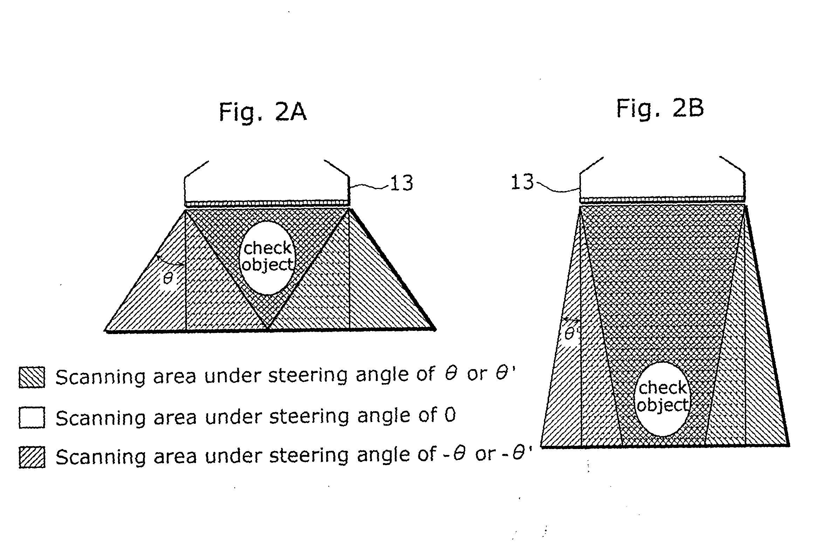

Fig. 2A shows how to scan a check object that is located shallowly, and Fig. 2B shows how to scan a check object that is located deeply. As shown in Fig. 2A and 2B, a check object must be positioned in the area that is partly the same as the scanning areas in the three steering angle directions when a check object is located deeply (when a check object is distant from an array ultrasound probe). In this case, it is impossible to eliminate speckle noises because the difference between angles becomes smaller and the appearing patterns of speckle noises do not change so much.

## SUMMARY OF THE INVENTION

The present invention aims at providing an image processing apparatus, a method and a program for eliminating speckle noises. Also, the present invention aims at providing an image processing apparatus, a method and a program for improving image quality by, for example, clearly displaying the edge of an image.

The image processing apparatus of the present invention is for sending ultrasound from an ultrasound probe and generating an image based on reflection signals generated from echoes from a check object, the image processing apparatus comprises: a specifying unit operable to specify at least two frequency bands in reflection signals; a generating unit operable to generate images for each of the specified frequency bands based on each of signals having the specified frequency bands in the reflection signals; and a synthesizing unit operable to synthesize the generated images for the respective frequency bands. With this structure, it is possible to generate a plurality of images from signals with different frequency bands that are contained in reflection signals, make a big difference in appearing patterns of speckle noises of the generated images for the respective frequency bands, further, synthesize these generated images. Therefore, it is possible to lessen the influence of speckle noises, that is, eliminate or reduce speckle noises.

Here, these units may be the following ones: the array ultrasound probe sends ultrasound at a plurality of steering angles, the specifying unit specifies different frequency bands for at least two steering angles in the plurality of steering angles, the generating unit generates images for the respective frequency bands based on signals in the specified frequency bands in reflection signals at the respective steering angles, and the synthesizing unit synthesizes the generated images for the respective steering angles. With this structure, it is possible to make a big difference in appearing pattern of speckle noises in the generated images obtained at the respective steering angles by changing the frequency band of the original reflection signals into another frequency band of the reflection signals, lessen the influence of speckle noises on the synthesized image, and eliminate or reduce speckle noises.

Here, the specifying unit may specify another frequency band as to the predetermined steering angle in a plurality of steering angles. With this structure, it can be realized in a way that it simply specifies another frequency band as to the specified steering angle.

Also, the specifying unit equips an association table for storing a plurality of sets including at least a first and a second sets, each of which is made of a steering angle group and a frequency band group associating with the plurality of steering angles, the image processing apparatus may control the steering angle of the ultrasound probe based on the steering angle group in one of the plurality of sets stored in the association table, the specifying unit may specify the frequency band to the respective steering angles based on the frequency band group associating with the steering angle group in the current set, and in the association table, a difference between frequency bands in the first frequency band group is designed to be bigger than a difference between frequency bands in the second frequency band group when a difference between steering angles in the first steering angle group is smaller than a difference between steering angles in the second steering angle group. With this structure, it is possible to make a big difference in appearing pattern of speckle noises by using widely different frequency bands even when the difference between steering angles is comparatively small, and eliminate or reduce speckle noises.

Here, the synthesizing unit may synthesize the "i" th pixel value f_g(i) of a synthesized image according to a pixel value calculation shown in formula 3, M being the number of the images for the respective steering angles generated by the generating unit, N being the number of pixels of the respective images, f_m(i) being an "i" th pixel value of a "m" th image in the generated images, and S being a value within a range of a possible f_m(i).

(formula 3)

$$f\_g(i)=S-M/((1/(S-f\_0(i)))+(1/(S-f\_1(i)))+...+(1/(S-f\_(M-1)(i))))$$

$$f\_g\ (i)=S-M/\ (\ (1/\ (S-f\_0\ (i)\ )\ )+(1/\ (S-f\_1\ (i)\ )\ )$$

$$+...\ +(1/\ (S-f\_\ (M-1)\ (i)\ )\ )\ )$$

These units not only eliminate or reduce speckle noises, but also eliminate the influence of values that are extremely different from value S of the standard value in a plurality of pixel values to be synthesized. It is possible to emphasize and display the part with pixel values around the standard value in the picture as a synthesized result.

Also, the image processing apparatus further comprises an area discriminating unit for discriminating the first area that includes the edges based on the images generated in the respective steering angle directions generated by the generating unit from the other parts. The synthesizing unit may perform the first synthesis operation as to the pixels in the first area of images generated in the respective steering angles that are generated by the generating unit and perform an operation different from the first synthesis operation as to the pixels in the areas except the first area. With this structure, it is possible to select an optimum pixel value operation method for the first area and other areas respectively, synthesize an image performing an optimum pixel value operation for the area including edges, the low luminance area and the other areas respectively, and display the image beautifully.

Also, the image processing method and the program of the present invention are made up to the similar structures as mentioned above.

As explained up to this point, the image processing apparatus of the present invention can eliminate or reduce speckle noises as its effect.

In addition to this, other effects are that it can eliminate the influence of values that are widely different from the standard value in a plurality of pixel values to be synthesized and emphasize and display the part with pixel values around the standard value in the image as the synthesis result.

Further, it can select the optimum pixel value operation method for the respective areas, synthesize images using the optimum pixel value operation for the respective areas such as the area including edges, the low luminance area and the other areas, and display the image beautifully.

Also, it can display edges or the neighboring part of an image beautifully.

Further, it can synthesize images according to the properties of the respective areas in the images.

Also, the image processing method and the program of the present invention has the same effect as mentioned above.

## FURTHER INFORMATION ABOUT TECHNICAL BACKGROUND TO THIS APPLICATION

filed , is incorporated herein by reference.

Japanese Patent application No. 2002-300397 filed October 15, 2002.

## BRIEF DESCRIPTION OF THE DRAWINGS

These and other subjects, advantages and features of the invention will become apparent from the following description thereof taken in conjunction with the accompanying drawings that illustrate a specific embodiment of the invention. In the Drawings:

Fig. 1A is a diagram showing how an ultrasound beam is irradiated from an array ultrasound probe in a plurality of directions.

Fig. 1B is a diagram showing a check object.

Fig. 1C is a diagram showing image data that are obtained in three directions.

Fig. 2A is a diagram showing how a check object that is located shallowly is scanned.

Fig. 2B is a diagram showing how a check object that is located deeply is scanned.

Fig. 3 is an external view of the ultrasound diagnosis apparatus in the first embodiment of the present invention.

Fig. 4 is a block diagram showing the main functional units of the ultrasound diagnosis apparatus.

Fig. 5 is a diagram showing a steering angle table and a pointer P.

Fig. 6A is a diagram showing a frequency band table and a pointer Q.

Fig. 6B is a diagram showing a pointer-associating table.

Fig. 7 is an illustration of a relation between steering angles, frequency bands and images to be generated.

Fig. 8 is another example illustration of a relation between steering angles, frequency bands and the generated images.

Fig. 9 is another example illustration of a relation between a steering angle, frequency bands and the generated images.

Fig. 10 is an illustration showing that appearing patterns of speckle noises vary depending on which frequency band is used.

Fig. 11 is a flow chart showing the processing for generating a synthesized image in the ultrasound diagnosis apparatus.

Fig. 12 is a block diagram showing the structure of the ultrasound diagnosis apparatus in the second embodiment of the present invention.

Fig. 13 is an example illustration of the generation method of mask data.

Fig. 14 is a flow chart showing the synthesizing operation by the image synthesizing unit.

Fig. 15 is a diagram showing the outline structure of the ultrasound diagnosis apparatus in the third embodiment.

Fig. 16 is a diagram showing an example of the processing flow when off-line.

## DESCRIPTION OF THE PREFERRED EMBODIMENT

(First Embodiment)

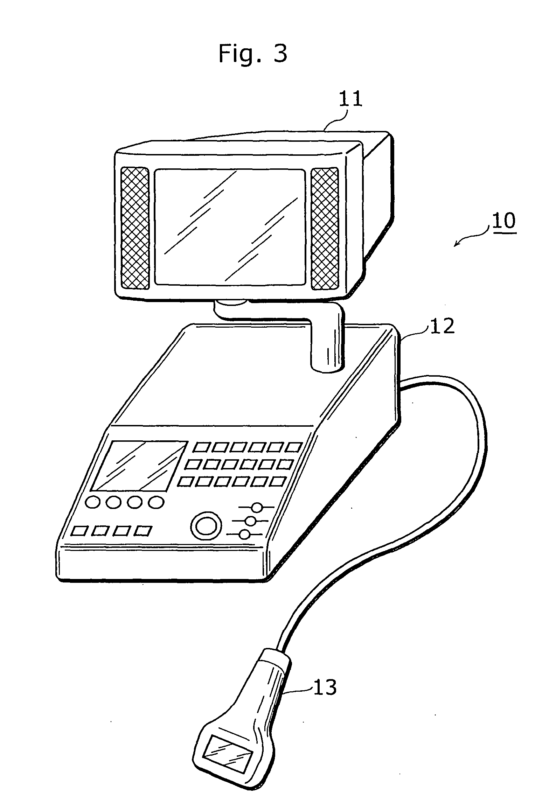

Fig. 3 is an external view of the ultrasound diagnosis apparatus 10 in the first embodiment of the present invention. This ultrasound diagnosis apparatus 10 is the one for eliminating speckle noises by generating images based on the reflection signals in the different frequency bands when displaying a tomography image by compound scan, and synthesizing these images and it comprises a display device 11, a body apparatus 12 and a probe 13 as its hardware structure.

The display device 11 is a CRT or the like, and it displays the obtained tomography image, edges, the measured result and the like in grey scale or in color. A transparent touch panel may be attached to the front of the CRT so as to obtain the user instruction on the displayed image via a touch pen.

The probe 13 is a probe that is composed of ultrasound transducers, acoustic lenses and the like for sending and receiving ultrasound.

The body apparatus 12 is a computer that is composed of a sending and receiving circuits for electronic scan by an ultrasound signal and an image processing circuit that are composed of a DSP or a CPU, and it comprises a group of switches for communicating with an operator, a trackball, an operation panel with a liquid crystal display unit and the like, a mouse and so on.

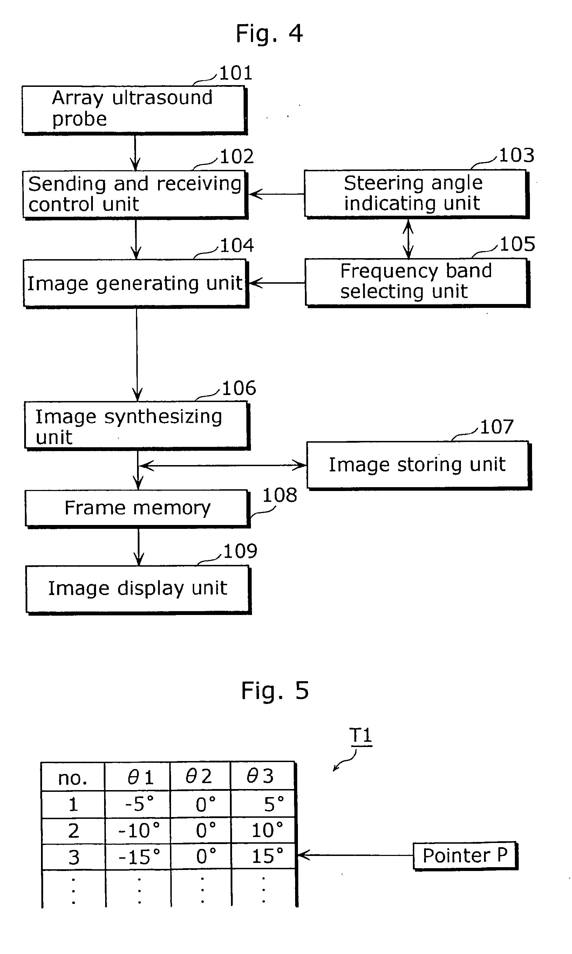

Fig. 4 is a block diagram showing the main functional units of the ultrasound diagnosis apparatus 10 shown in Fig. 3. This ultrasound diagnosis apparatus 10 comprises an array ultrasound probe 101, a sending and receiving control unit 102, a steering angle specifying unit 103, an image generating unit 104, a frequency band selecting unit 105, an image synthesizing unit 106, an image storing unit 107, a frame memory 108 and an image display unit 109.

The array ultrasound probe 101 has the function of the probe 13 shown in Fig. 3, and it receives control information from the sending and receiving control unit 102 and then actually sends and receives the sound waves. At that time, the array ultrasound probe 101 is electronically steerable as shown in Fig. 1A by adjusting the delay amount when sending and receiving sound waves based on the instruction from the sending and receiving control unit 102. A linear array probe is shown here, but a convex array probe is also available. A steering angle can be set by the input from the steering angle specifying unit 103. This steering function makes it possible to obtain data concerning the same subject that is observed in different angle directions.

The sending and receiving control unit 102 controls sending and receiving sound in the array ultrasound probe 101 and controls the steering angle according to the instruction by the steering angle indicating unit 103.

The steering angle specifying unit 103 specifies a plurality of steering angles in compound scan to the sending and receiving control unit 102.

For example, the steering angle specifying unit 103 holds the steering angle table T1 shown in Fig. 5 and the pointer P inside, and it specifies a plurality of steering angles in order according to the entry that is pointed by the pointer P. The steering angle table T1 stores the entry numbers (which is no. in the figure) and a steering angle group (a plurality of steering angles θ1, θ2 and θ3) associating with each other. The steering angle group is set so that the difference between steering angles becomes biggest in entry No. 1, becomes smaller in entry No. 2, and becomes smallest in entry No. 3 in the steering angle table T1 in the same figure. The entry numbers that are hold by the pointer P is selectively set by a default value or an operator.

The image generating unit 104 performs an amplification operation or an interpolation operation on signals having the frequency band selected by the frequency band selecting unit 105 in signals that are contained in sound signals that are received in the respective steering angle directions according to the control by the sending and receiving control unit 102 and generates an image.

The frequency band selecting unit 105 selects the frequency bands for the respective steering angles that are specified by the steering angle specifying unit 103 and specifies the frequency bands to the image generating unit 104. At that time, the frequency band selecting unit 105 selects at least two different frequency bands so as to make the image generating unit 104 generate images based on the signals having a different frequency band in reflection signals and make a difference in appearing patterns of speckle noises resulting from the difference in frequent bands.

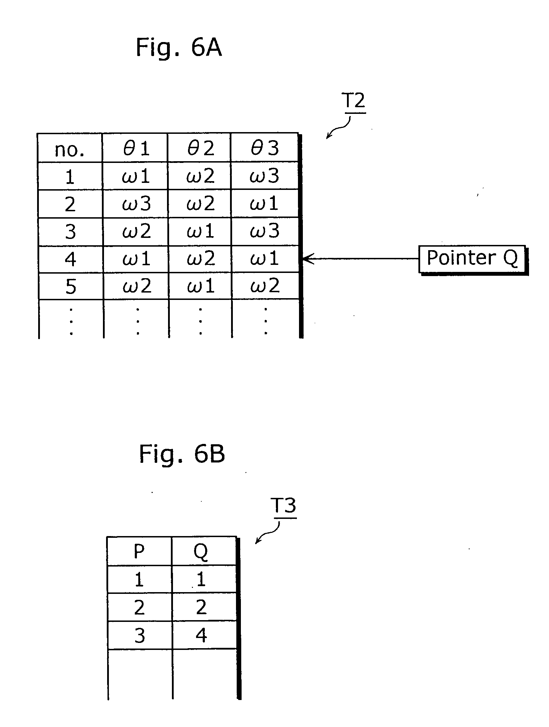

As a specific structural example, the frequency band selecting unit 105 holds the frequency band table T2 and the pointer Q shown in Fig. 6A and a pointer-associating table T3 shown in Fig. 6B inside, and it specifies frequency bands in order according to the entry that is pointed by the pointer Q. This frequency band table T2 stores the entry numbers (which is no. in the figure) and a frequency band group of (a plurality of frequency bands $\omega1$, $\omega2$ and $\omega3$) associating with each other. Here, $\omega1$ is a basic wave band with the same frequency as the sound signal that is sent from the array ultrasound probe 101, $\omega2$ is the second harmonics band whose frequency is twice the basic wave, and $\omega3$ is the third harmonics band whose frequency is three times the basic wave.

The pointer-associating table T3 is a table for associating the pointer P with the pointer Q. In this example, pointer P and pointer Q are associated with each other so that a steering angle group with a small difference (no. 1 in the entry in Fig. 5) is associated with a frequency band group with a big difference (no. 1 in the entry in Fig. 6). In other words, providing that the entry no. 1 in Fig. 5 and the entry no. 1 in Fig. 6 are made to be a pair after association by the pointer-associating table T3, when the difference between steering angles of the first pair (5°) (no. 1 in Fig. 5 and no. 1 in Fig. 6) is smaller than the difference between steering angles of the second pair (15°) (no. 3 in Fig. 5 and no. 4 in Fig. 6), the difference between frequency bands of the first frequency band group (entry no. 1 in Fig. 6) is set to be bigger than the difference between frequency bands of the second frequency band group (entry no. 4 in Fig. 6). The above setting is made so as to make a big difference in appearing pattern of speckle noises by making a big difference in frequency band since it is considered that the difference in appearing pattern of speckle noises becomes smaller as the difference between steering angles gets smaller.

The image synthesizing unit 106 synthesizes images that are generated by the image generating unit 104 for the respective steering angles by, for example, weighting and arithmetic averaging pixel values that are located in the same position and stores the pixel value average as the pixel value of the synthesized image in the image storing unit 107 and the frame memory 108.

The image storing unit 107 is equipped so as to read out the synthesized image when off-line and display the image on the image display unit 109 via the frame memory 108.

The frame memory 108 is for storing the synthesized image obtained from the image synthesizing unit 106 when real time processing or the synthesized image from the image storing unit 107 when off-line.

The image display unit 109 corresponds to the display device 11 shown in Fig. 3.

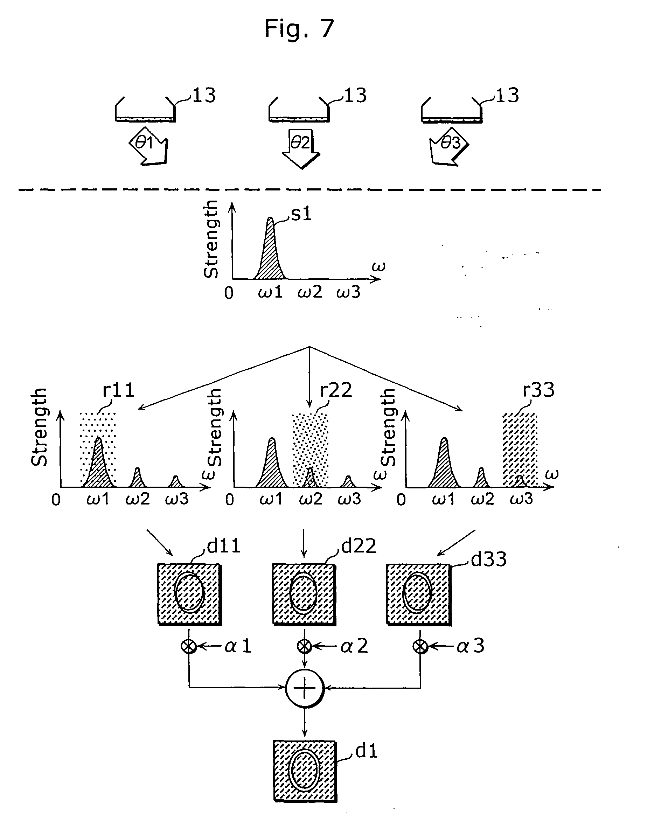

Fig. 7 is an illustration of relations between the steering angles that are specified by the steering angle specifying unit 103, frequency bands that are selected by the frequency band selecting unit 105, and images generated by the image generating unit 104.

The upper-most column (the first column) in the same figure shows the steering angles that are sent from the array ultrasound probe 101 (probe 13). In reality, the array ultrasound probe 101 is fixed at one point and sends sound wave in three directions, but three array ultrasound probes 101 are shown for the respective three directions for convenience.

The second column shows frequency band $\omega1$ (basic wave) of the sent wave s1 from the array ultrasound probe 101.

The third column shows that the image generating unit 104 generates images d11, d22 and d33 using the signal r11 having the frequency band $\omega1$ for the reflection signal at steering angle $\theta1$, the signal r22 having the frequency band $\omega2$ for the reflection signal at steering angle $\theta2$, and the signal r33 having the frequency band $\omega3$ for the reflection signal at steering angle $\theta3$ respectively. This corresponds to the case where the frequency band as the entry no. 1 in the frequency table T2 shown in Fig. 6A is specified.

The fourth column shows the generated three images r11, r22 and r33. The appearing patterns of speckle noises in these three images widely differ from each other because both the steering angles and the frequency bands differ from each other.

The fifth column shows the synthesized result of these images d1. In this way, it is possible to sufficiently eliminate speckle noises. In other words, it is possible to eliminate or reduce speckle noises in the synthesized image because the appearing patterns of speckle noises on these three generated images widely differ from each other. Also, this synthesis is made by performing arithmetic averaging taking the weighing coefficients $\alpha1$, $\alpha2$ and $\alpha3$ as to the respective pixels in the same position of these three images.

Here, $\alpha1: \alpha2: \alpha3$ may be, for example, 1:10:100. It is because the amplitude ratio of the basic wave, the second harmonics and the third harmonics that are contained in the reflection signal is about 100:10:1.

Also, when providing that $\alpha1: \alpha2: \alpha3$ is 1:1:1, a contour contrast component of harmonics component is added to the image of basic wave although speckle noises are not sufficiently eliminated.

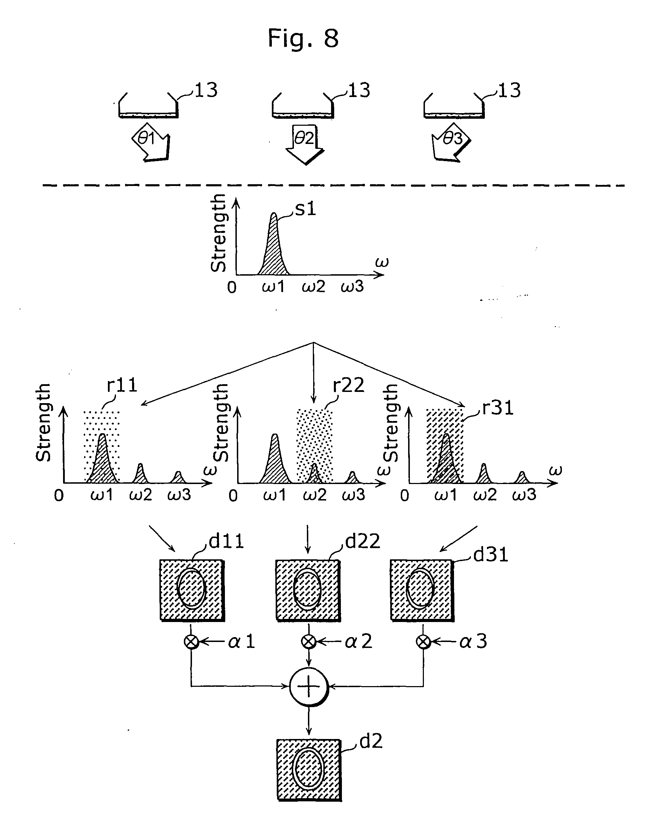

Fig. 8 is another example illustration of relations between steering angles, frequency bands and generated images. The same figure differs from Fig. 7 in that the frequency band corresponding to the steering angle $\theta3$ is not $\omega3$, but $\omega1$.

The two images r11 and r31 that are generated from the echoes obtained in the directions of steering angles $\theta1$ and $\theta3$ in the three images r11, r22 and r31 that are generated by the image generating unit 104 are obtained using the same frequency band $\omega1$. In this case, there is a big difference in appearing patterns of speckle noises because the difference between steering angles (the difference between angle $\theta1$ and angle $\theta3$) is big. As to the two images r11 and r22 that are generated from the echoes obtained in the directions of steering angles $\theta1$ and $\theta2$, the difference

between steering angles (the difference between angle θ1 and angle θ2) is not big, but the difference in appearing pattern of speckle noises is big because two kinds of frequency bands ω1 and ω2 are used to generate r11 and r22 respectively. The same thing is true of the two images r22 and r31 that are obtained in the directions of steering angles θ2 and θ3.

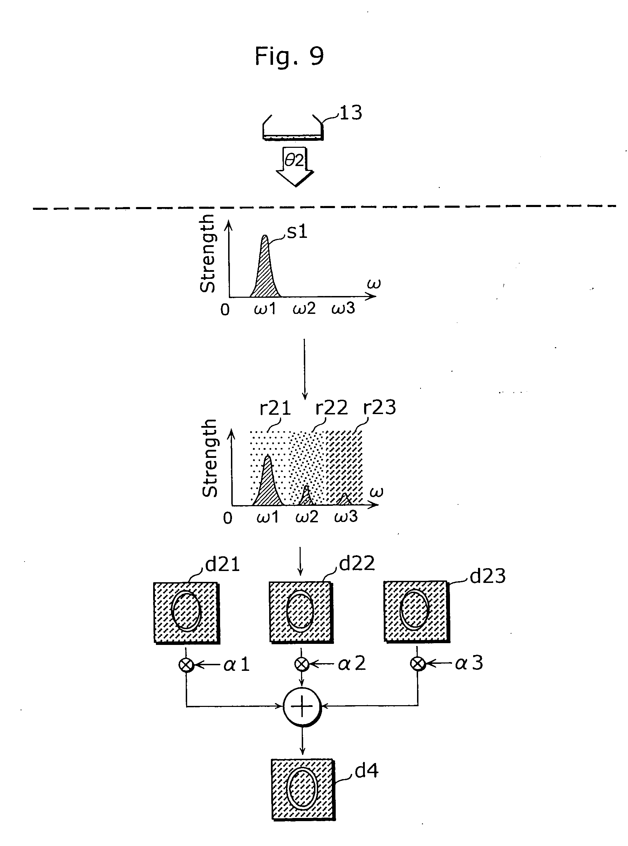

Fig. 9 is another example illustration of a relation between a steering angle, a frequency band and generated images. The same figure differs from Fig. 7 and Fig. 8 in that the steering angle of the sent wave is not three, but one. In other words, the sent wave s1 is sent in the direction of steering angle θ2 only.

In the fourth column of the same figure, the three images d21, d22 and d23 that are generated from the echoes obtained in the direction of steering angle θ2 in the three images r21, r22 and r23 that are generated by the image generating unit 104 generated using the same steering angle, but the appearing patterns of speckle noises differ from each other because three kinds of frequency bands ω1, ω2 and ω3 are used. In this way, it is possible to reduce speckle noises when using different frequency bands as shown in Fig. 9 although it is possible to reduce speckle noises more when a different steering angle and a frequency band are concurrently used for the respective images as shown in Fig. 7 and Fig. 8.

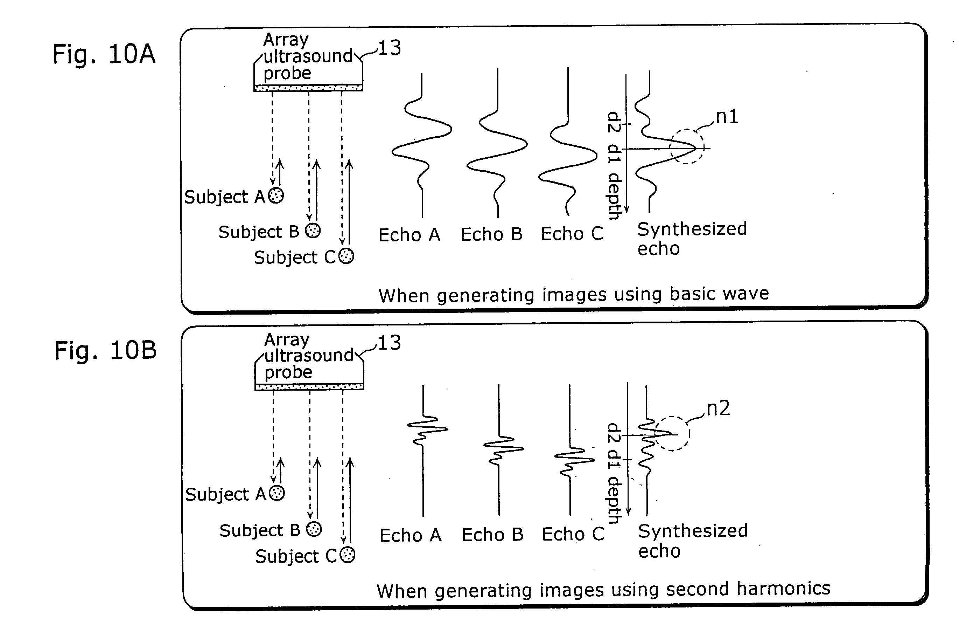

Fig. 10A and Fig. 10B are illustrations of the difference in appearing pattern of speckle noises when different frequency bands are used. Fig. 10A schematically shows what the echoes in the basic wave band look like. Subject A, B and C in the figure are located at the approximately same interval as the wavelength of the basic wave. The vertical axes in a downward direction of the respective waveforms show the distances (depth "d"s) from the array ultrasound probe 101 to the subject and the horizontal axes show the luminance. The respective echoes A, B and C from subject A, B and C show the respective waveforms on assumption that the array ultrasound probe 101 received only echoes from the current subjects. The synthesized echo shows the waveform used as the received wave so as to generate an image.

Likewise, Fig. 10B schematically shows what the second harmonics (echoes) look like.

Echoes that are synthesized in Fig. 10A and 10B have high luminance parts n1 and n2 to be stochastically generated by influence, and these high luminance components become a cause of speckle noises.

However, the position of the subject depth d1 of the echoes synthesized in the basic wave band in Fig. 10A is different from that of the subject depth d2 of the echoes synthesized in the second harmonics band in Fig. 10B. Consequently, the distribution pattern of the speckles (or the way the variable components appear) in the images generated using echoes in the basic wave band is widely different from that of the speckles in the images generated using echoes in the second harmonics band. Therefore, when synthesizing these generated images, it is possible to reduce the influence of variable components (regard the variable components as white noises).

The functions of the ultrasound diagnosis apparatus 10 in the first embodiment of the present invention that is formed as mentioned up to this point will be explained.

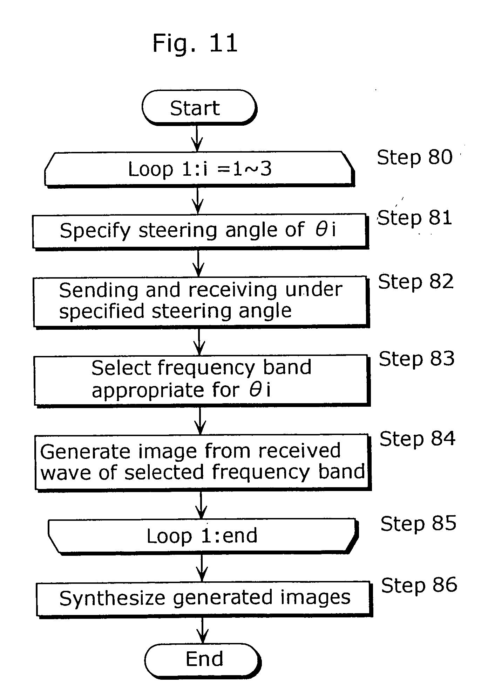

Fig. 11 is a flow chart showing the processing up to generating a synthesized image, that is, the processing shown in Fig. 6 and 7, in the ultrasound diagnosis apparatus 10.

As the number of times the loop 1 (steps 80 ~ 85) is repeated is the same as the number of steering angles used for scanning in the same figure, one processing will be explained. When the steering angle indicating unit 103 reads out the steering angle θi from the steering angle table T1 (Fig. 5) and specifies the steering angle to the sending and receiving control unit 102 (step 81), the sending and receiving control unit 102 sends ultrasound using the steering angle by controlling the array ultrasound probe 101, receives the echoes (step 82), and outputs the received signal to the image generating unit 104. At that time, the frequency band selecting unit 105 specifies a frequency band ω to the image generating unit 104 by reading out and selecting the frequency band ω that corresponds to θ i from the frequency table T2 (Fig. 6A)(step 83). The image generating unit 104 generates an image based on the signal having the frequency band specified by the frequency band selecting unit 105 in the signals received from the sending and receiving control unit 102 (step 84). Images are generated based on the signals having the frequency band specified in echoes by the frequency band selecting unit 105 respectively for the steering angles specified by the steering angle indicating unit 103 by performing this loop 1 processing.

As explained up to this point, the ultrasound diagnosis apparatus in this embodiment can make a big difference in appearing pattern of speckle noises on the images to be generated for the respective steering angles, reduce the influence of speckle noises in the synthesized image, and eliminate or reduce the speckle noises by making a difference in frequency band for the respective steering angles considering the principle of appearance of speckle noises.

Note that the steering angle indicating unit 103 may be formed in a way that it specifies a different frequency band when using a predetermined steering angle in a plurality of steering angles or in a way that it receives a user input. In this way, as to the predetermined steering angle, it can be realized in a simple way that it specifies a different frequency band. This is true of the frequency band selecting unit 105.

Also, the number of the steering angles when scanning are three here, but the number is not limited to three. The number of the steering angles when scanning is determined based on the number of steering angles θ1, θ2, θ3 ... in the steering angle table T1. The desirable number of steering angles may be written in this steering angle table T1.

In addition to this, the same number of frequency bands may be written in the frequency band table T2.

Also, as shown in Fig. 9, it is also possible to generate respective images from the received waves of these different frequency bands that are contained in echoes at a single steering angle and synthesize the generated images.

Further, the image generating unit 104 may be another form in a way that it generates images after weighing signals in the respective frequency bands, while a form where the image synthesizing unit 106 synthesizes the images generated by the image generating unit 104 taking weighing coefficient $\alpha 1$, $\alpha 2$ ... is explained in the above-mentioned embodiment. At that time, the weighing coefficients may be the values that are inversely proportional to the signal strength for the respective frequency bands like the above-mentioned $\alpha 1$, $\alpha 2$ ... .

(Second Embodiment)

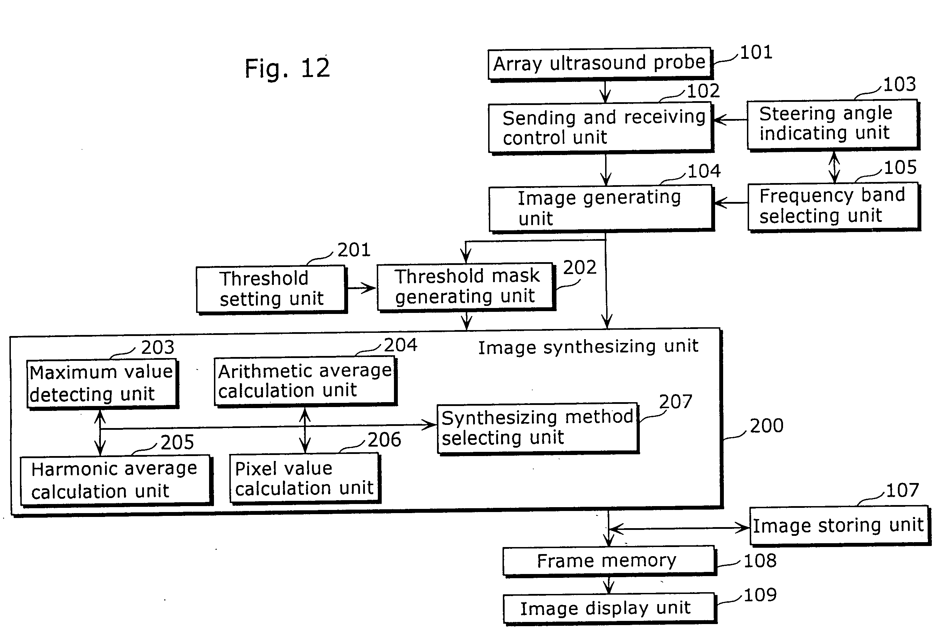

Fig. 12 is a block diagram showing the structure of the ultrasound diagnosis apparatus in the second embodiment of the present invention. The apparatus differs, compared to the form shown in Fig. 4, in that a threshold setting unit 201 and a threshold mask generating unit 202 are newly added, and that an image synthesizing unit 200 is equipped instead of an image synthesizing unit 106.

This embodiment makes it possible to display the area including edges in an image more beautifully by introducing an operation method for synthesizing images in a way of emphasizing pixel values around the pixel values to be edges and by making it possible to select either one of operation methods depending on whether the area includes edges or not, while the image synthesizing unit 106 in the first embodiment synthesizes images by arithmetic averaging. As the units in Fig. 12 to which the same reference numbers as the ones in Fig. 4 are assigned have the same functions, the other units will be mainly explained.

The image generating unit 200 comprises a maximum value detecting unit 203, an arithmetic averaging calculation unit 204, a harmonic average calculation unit 205, a pixel value calculation unit 206 and a synthesis method selecting unit 207 so as to become capable of selecting either one of operation methods depending on whether the area includes edges or not in a plurality of synthesis operation methods, while the image synthesizing unit 106 in the first embodiment synthesizes images by arithmetic averaging.

The maximum value detecting unit 203 detects the maximum value as to the corresponding pixels in a plurality of image data generated by the image generating unit 104. In other words, the maximum value detecting unit 203 focuses on the pixel values that are located in the same position in a plurality of image data, selects the one which has the biggest pixel value, and regards it as the pixel value of the corresponding position in the synthesized image. This method is effective in that it makes it possible to form subjects at higher luminance when it is obvious that signals for forming subjects exist in the position.

The arithmetic average calculation unit 204 obtains the arithmetic average as to the corresponding pixels of a plurality of data generated by the image generating unit 104, and regards the arithmetic average as the pixel value of the pixels in the synthesized image. The arithmetic average is the most generally used method when generating a synthesized image by compound scan. The pixel value f_g (i) of a synthesized image by arithmetic averaging is shown by the following formula:

$$f\_g\ (i) = (\ f\_0\ (i) + f\_1\ (i) + ... + f\_\ (M\text{-}1)\ (i))\ /\ M$$

where, the number of the image data to be synthesized (the number of its pixel value is N) is M, the "i" th ("i" is an integer of $1 \sim$ N) pixel value in the "m" th ("m" is an integer of $0 \sim$ (M-1)) image data is f_m(i), and the "i" th pixel value of the synthesized image is f_g(i).

In the image synthesized by arithmetic averaging, pixel values of a plurality of images are evenly reflected on the pixel values of the synthesized image, and apparently natural synthesis result is obtainable. Also, a low pass filter (LPF) effect is obtainable by the overlapping effect, which makes it possible to reduce the influence of the general white noises that is accompanied by the Gaussian distribution by performing this processing. However, solely using this method is undesirable because blur also occurs on the edge of the subject should be clearly formed, while the same effect is expectable when the appearing patterns of speckle noises are different among a plurality of image data.

The harmonic average calculation unit 205 obtains the harmonic average as to the corresponding pixels in a plurality of image data generated by the image generating unit 104, and regards the harmonic average as the pixel value of the pixel in the synthesized image. The pixel value f_g(i) in the synthesized image by harmonic averaging is shown by the following formula:

$$f\_g\ (i) = M\ /\ (\ (\ 1\ /\ f\_0\ (i)\ ) + (\ 1\ /\ f\_0\ (i)\ ) + ... + (\ 1\ /\ f\_(M\text{-}1)\ (i)\ )\ )$$

As it is impossible to calculate harmonic average when 0 or a negative value exists as a pixel value, a processing of switching pixel value 0 to a positive value excluding 0 (for example, 1) or the like is performed. Also, if there exists any negative value that is not generally included in the pixel values, a processing of switching pixel value 0 to a positive value excluding 0 or the like is performed. The harmonic average makes it possible to reduce the influence on this value $f\_g(i)$ when an extremely different value (a big value) exists.

As to the corresponding pixels in a plurality of image data generated by the image generating unit 104, the pixel value calculation unit 206 calculates the pixel value of the synthesized image according to the following formula $f\_g(i)$ and regards the pixel value as the pixel value of the pixel in the synthesized image.

$$f\_g(i) = S\text{-}M / ( (1 / (S\text{-}f\_0(i))) + (1 / (S\text{-}f\_1(i))) + ... +$$

$$(1 / (S\text{-}f\_(N\text{-}1)(i))))$$

Here, S is a standard value that has the value below the possible $f\_m(i)$ value. In other words, this pixel value close to S is reflected so as to be emphasized in $f\_g(i)$. As a positive value except S is needed as $f\_m(i)$ when performing this pixel calculation, when the $f\_m(i)$ is equal to S, the value is replaced by a positive value close to S except S (for example, S+1).

As to the images generated by the image generating unit 104 for the respective steering angles, the synthesizing method selecting unit 207 selects either one of the four calculation units 203 ∼ 206 mentioned above in the case of the pixels in the area including edges so as to make the unit perform calculation or selects another one of the four calculation units 203 ∼ 206 in the case of the pixels in the other areas so as to make the unit perform calculation. Whether the area includes edges or not is judged based on the mask data generated by the threshold setting unit 201 and the threshold mask generating unit 202. In other words, the synthesizing method selecting unit 207 selects an image synthesizing method in the methods for the respective pixels performed by the calculation units 201∼204 mentioned above based on the mask data generated by the threshold mask generating unit 202.

The threshold setting unit 201 sets thresholds as to the respective images generated by the image generating unit 104. There are several threshold setting methods as follows: ( i ) a user sets an arbitrary value using an input device (such as a keyboard, a trackball and a switch) that belongs to an ultrasound diagnosis apparatus, ( ii ) the value predetermined based on a parameter such as the frequency of a sound signal, the amplitude rate when generating an image, the used frequency band, the synthesizing method and so on, ( iii ) after an arbitrary area of the image data to be synthesized is specified, either one of pixel values is set as the threshold, they are, for example, the average value, the middle value, the maximum value, the minimum value or the like in the pixel values in the area.

The threshold mask generating unit 202 judges the size of a pixel value of image data based on the threshold held in the threshold setting unit 201 and generates mask data including the results. The mask data here shows whether the pixel exceeds the threshold or not and the threshold is set as the value that shows the limit of the pixel value of an edge or the neighboring part of an edge in an image.

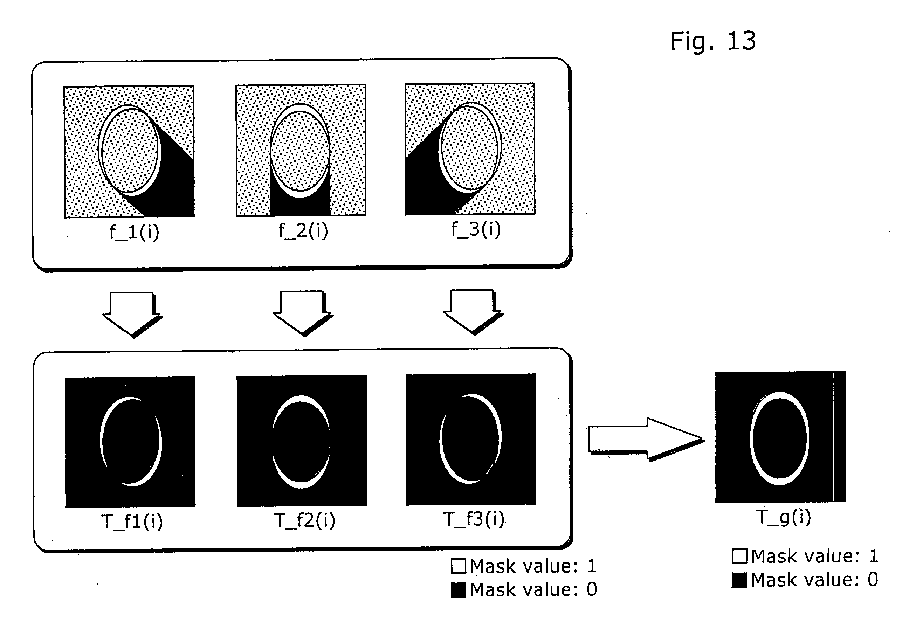

Fig. 13 is an illustration of an example of mask data generating methods.

Here, only a single threshold Th is given, and the threshold mask generating unit 202 generates a synthesized image from three image data ($f\_1$, $f\_2$ and $f\_3$) that have "N" pieces of pixel values.

When the pixel $f\_1(i)$ of the image data $f\_1$ (i is 1 ∼ N) is bigger than Th, the mask data on this image data is $T\_f1(i) = 1$. This means that the pixel exists on an edge or the neighboring part of an edge. When it is below Th, $T\_f1(i) = 0$. This means that the pixel exists on the other areas. The threshold mask generating unit 202 performs this processing on all the pixel values and three image data, and it generates respective mask data $T\_f1$, $T\_f2$ and $T\_f3$ mentioned in the upper column of the Fig. 13.

Further, as shown in the lower column of the Fig. 13, the threshold mask generating unit 202 generates the mask data $T\_g(i)$ that is used for synthesizing images from three mask data $T\_f1$, $T\_f2$ and $T\_f3$. In the three mask data in the same figure, mask values are 1 (white part) or 0 (black part). Here, the mask data for synthesis is the logical OR of these three mask data.

As this is an example, the threshold mask data for synthesis may be obtained by logical product or a multi-valued mask data that have mask values except 0 and 1 after increasing the number of bits per one pixel of mask data when an image where all the image data are above the threshold is needed. In this case, over three areas such as an edge area, its neighboring area, a low luminance area and the like are distinguishable.

The operations of the ultrasound diagnosis apparatus in the embodiment that is formed in this way will be explained.

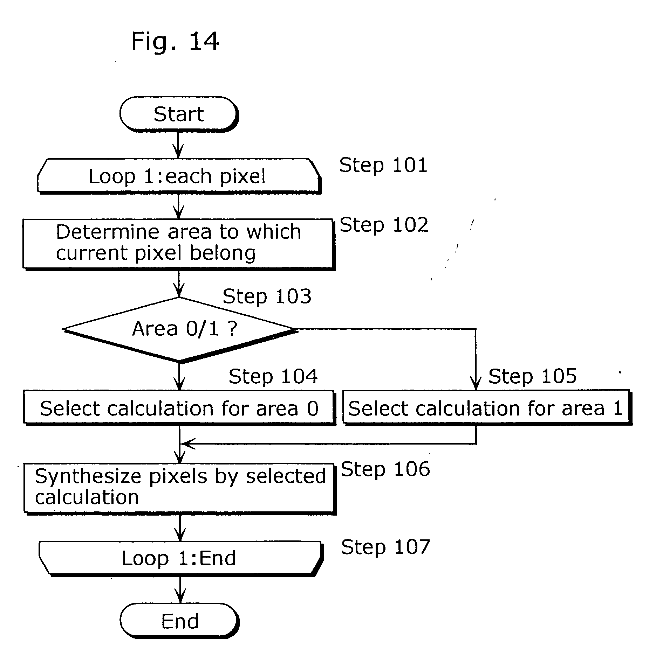

Fig. 14 is a flow chart showing the synthesizing operations of the image synthesizing unit 200. In the same figure, as the number of times the loop 1 (steps 101 ∼ 107) is repeated is the same as the number of pixels of the image N that is generated by the image generating unit 104, one processing will be explained below.

The synthesizing method selecting unit 207 judges whether the mask data T _g (i) that corresponds to the pixels to be synthesized equals 1 (whether the area is area 1 including edges) or not and whether the mask data T _g (i) equals 0 (whether the other area is area 0)(step 102), selects the operation for area 1 (step 105) or the operation for area 0 (step 104) according to the judgment result (step 103), and makes either one of calculation units 203 ~206 appropriate for handling the selected operation perform synthesis operation on the pixels (step 106).

In this way, the synthesizing method selecting unit 207 selects a synthesizing method based on the value of the threshold mask data T _g (i) generated by the threshold mask generating unit 202. In other words, for example, arithmetic averages of f _1 (i), f _2 (i) and f _3 (i) is performed in the T _g (i) = 0 area and a maximum value detection is performed in the T _g (i) = 1 area when T _g (i) is made of the two values of 0 and 1.

Also, as a high luminance value showing the existence of the check object exists in the place i when T _g (i) = 1, it becomes possible to form the subject more clearly by selecting the highest luminance in the three image data f _1 (i), f _2 (i) and f _3 (i). When T _g (i) = 0, there is a high possibility that the luminance value of the place i shows noises because the place i is not the edge part of the subject. When performing arithmetic average processing on the signal like this, LPF effect is obtainable and thus it is possible to reduce the noise components in the synthesized image.

Also, when the threshold mask data T _g (i) includes any value except 0 and 1 (when over three kinds of values are included), a synthesizing method is selectable for the respective values.

Here, how each calculation unit is selected will be elaborated on.

In the case of harmonic average processing, it is possible to reduce the influence of an extremely different (big) value in the series of data.

As the first example, when there is a group of data 80, 90, 100 and 1000, here are calculation results: 317.5 when selecting the arithmetic average calculation unit 204, 115.6 when selecting the harmonic average calculation unit 205 and 88.0 on condition that S = 75 when using the pixel value calculation unit 206.

As the second example, when there is a group of data 80, 90, 100 and 1, here are calculation results: 67.8 when selecting the arithmetic average calculation unit 204, 3.869 when selecting the harmonic average calculation unit 205 and 88.6 on condition that S = 75 when using the pixel value calculation unit 206.

Three values exist around 90 in the respective groups of data of the above-mentioned two examples, and 1000 or 1 is set as a widely different value. In other words, when comparing the above calculation results on condition that a value around 90 is approximately correct value, the calculation results obtained when using the arithmetic average calculation unit (204) are excessively far from 90 affected by the value 1000 or 1.

In the case of selecting the harmonic average calculation unit 205, it is possible to obtain a good result in the first example group of data 80, 90, 100 and 1000, but in the second example group of data 80, 90, 100 and 1, the calculation result is excessively far from 90 because the value around 1 heavily affects the calculation result obtained in the harmonic averaging as its property.

However, as shown in the second embodiment, it is possible to avoid processing the pixel value around 1 because a threshold is set so as to narrow the range of pixel values to be processed.

In the case of using pixel value calculation unit 206, the outputted calculation results of the respective data groups are close. It is because 75 near 90 is selected as the standard value S. In this formula, harmonic average of pixel values is obtained by reversing the relation between the big pixel value and the small pixel value and then the inverse processing of the calculation result is performed. Therefore, the formula of the pixel value calculation unit 206 is a variation for emphasizing the pixel value close to the standard value S preventing the pixel value close to 1 (the standard value) from being emphasized as its property of the harmonic averaging.

A user can freely select a synthesizing method. Also, the standard value S when using the pixel value calculation unit 206 can be arbitrary determined. When the luminance value that forms the check object can be quantitatively obtainable, it becomes possible to eliminate the influence of the widely different value by regarding the value as the standard value S and reduce the graininess in the image.

As explained up to this point, the ultrasound diagnosis apparatus in this embodiment makes it possible to emphasize and display the parts with pixel values around the standard value in the synthesized image by setting the same value as the pixel value to be emphasized as the standard value S in the pixel value calculation unit 206.

In this second embodiment, eliminating and reducing speckle noises in the first embodiment, realized by the pixel value calculation unit 206 produced a synergistic effect of improving image quality, and the effect of emphasizing edges realized by the pixel value calculation unit 206 is obtainable independently from the effects in the first embodiment. In other words, when presetting the pixel value showing an edge as the standard value S, it is possible to emphasize and display the area including edges in the synthesized image. Also, presetting the pixel values of the predetermined areas to be emphasized except the edge areas as the standard value S, it is possible to improve the image quality by emphasizing the current areas and display the areas beautifully.

Also, the threshold mask generating unit 202 can generate the mask data showing the area including edges, the low luminance area or the like, that is, it can discriminate which area it is by setting one, two or more values to be borders of a plurality of areas as the thresholds in the threshold setting unit 201. Further, the synthesizing method selecting unit 207 selects either one of calculation units 203~206 for the respective areas shown in the mask data and selects the optimum pixel calculation method for the respective areas, thus it is possible to synthesize the areas including edges, the low luminance area or the other areas using the optimum pixel calculation method for the respective areas and display the synthesized image beautifully. In addition to eliminating or reducing speckle noises in the first embodiment, selecting pixel calculation methods for the respective areas realized by the threshold setting unit 201 and the threshold mask generating unit 202 produces a synergistic effect of obtaining more beautiful images, and the effect obtained from selecting pixel calculation methods for the respective areas is obtainable independently from the effects in the first embodiment.

Also, the maximum value detecting unit 203 is desired to adjust images by, for example, leveling the average pixel levels as to a plurality of image data generated by the image generating unit 104. It is because each frequency band of a reflection signal, that is, a basic wave, the second harmonics, the third harmonics has a different strength (amplitude) of the received signal. In other words, the ratio of the amplitudes of the basic wave, the second harmonics and the third harmonics that are contained in the reflection signals are about 100:10:1, which means it is desirable to level the image data so as to equalize the respective values in this ratio of amplitude to each other. For example, the maximum value detecting unit 203 performs a weighing on the respective image data inversely proportional to the strengths of the received signals, levels the image data, and detects the maximum value in the image data after the leveling.

The arithmetic average calculation unit 204, the harmonic average calculation unit 205 or the pixel value calculation unit 206 can also level image data in the same way. In other words, the arithmetic average calculation unit 204 can be the unit calculating the pixel value f _g (i) of the synthesized image performing the arithmetic averaging plus a weighing like the image synthesizing unit 106 in the first embodiment. In this case, the arithmetic average calculation unit 204 calculates the pixel value f _g (i) of the synthesized image. In the following formula, the weighing coefficient to the "m" th ("m" is 0 ~ (M-1) ) image data is $\alpha$m.

$$f\_g(i)=(\alpha\_0 \cdot f\_0 \,(i) + \alpha\_1 \cdot f\_1(i)+...+ \alpha\_(M-1) \cdot f\_(M-1)(i))/M$$

Also, the harmonic average calculation unit 205 can be the unit calculating the pixel value f _g (i) of the synthesized image by performing the harmonic averaging plus a weighing as shown in the following formula.

$$f\_g(i)=M/((1/(\alpha\_0 \cdot f\_0 \,(i))+1/(\alpha\_1 \cdot f\_1(i))+...+1/(\alpha \_(M-1)\cdot$$

$$f\_(M-1)(i)))$$

Further, the pixel value calculation unit 206 can be the unit calculating the pixel value f _g (i) of the synthesized image performing the pixel calculation plus a weighing as shown in the following formula.

$$f\_g(i)=S-M/(p\_0(i)+p\_1(i)+...+p\_(M-1)(i)$$

$$p\_0(i)=1/(S- \alpha\_0 \cdot f\_0 \,(i))$$

$$p\_1(i)=1/(S-\alpha\_1 \cdot f\_1 \,(i))$$

$$p\_(M-1)(i)=1/(S-\alpha\_(M-1) \cdot f\_(M-1)(i))$$

Note that it is needless to perform a weighing in the image synthesizing unit 200 in the case where the image generating unit 104 performs a weighing using the above-mentioned weighing coefficient $\alpha$ and generates the image data.

(Third Embodiment)

A method for generating a higher quality synthesized image when off-line after storing the RF (Radio Frequency) signals that are the basis for generating image data will be explained in this third embodiment.

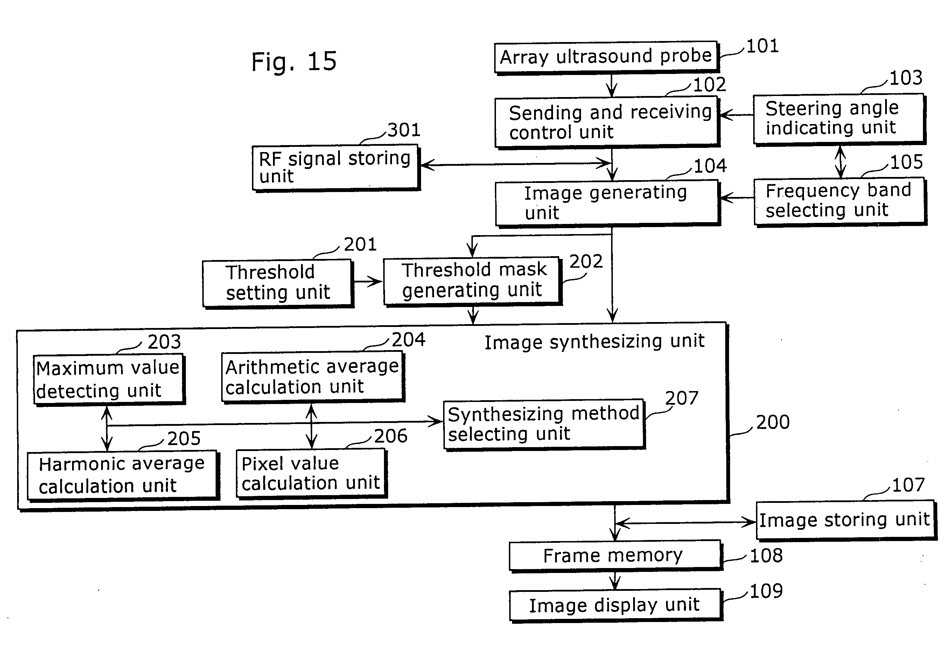

Fig. 15 shows the structural outline of the ultrasound diagnosis apparatus in the third embodiment as an applicable example of the present invention. The apparatus differs from the structure shown in Fig. 12 in that an RF signal storing unit 301 is newly added and the image generating unit 104 generates images using a plurality of frequency band (for example, in the above-mentioned ω1, ω2 and ω3) respectively in the respective steering angle directions when off-line. The different points are mainly focused on the following explanation.

The RF signal storing unit 301 is for storing the RF signals received by the sending and receiving control unit 102, and its capacity is enough to store the RF signals needed for synthesizing at least one synthesized image when off-line.

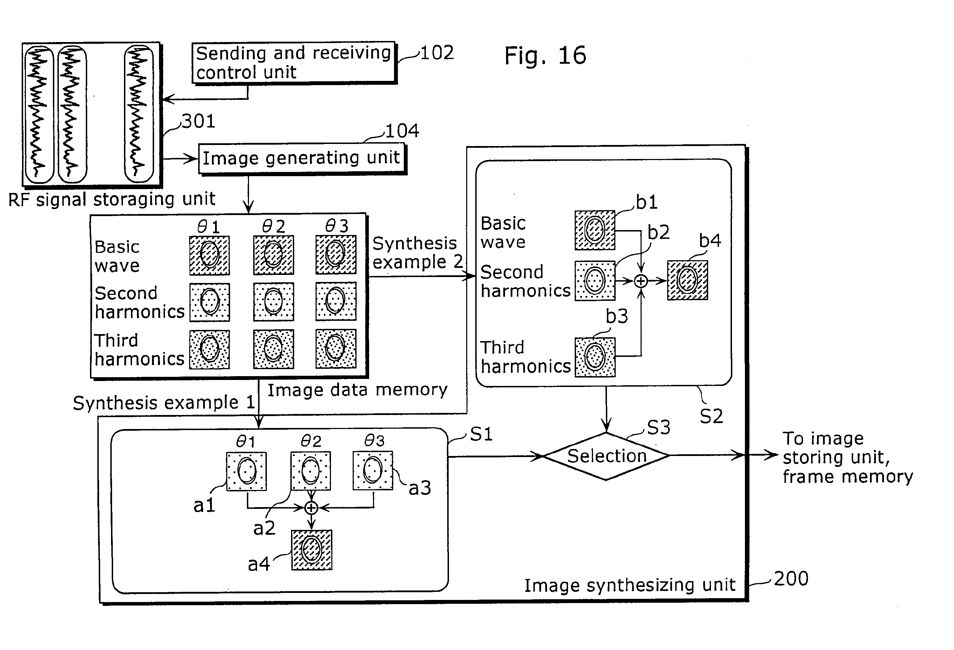

Fig. 16 is a diagram showing an example of the processing flow when off-line. The RF signals (that is, the received echoes) from the array ultrasound probe 101 are stored in the RF signal storing unit 301 when off-line. All the information needed for synthesizing an image is stored in the RF signal storing unit 301 when off-line.

The image generating unit 104 reads out the RF signals from the RF signal storing unit 301, generates the image data by changing various kinds of parameters (a frequency band and so on), and stores the image data memory (not shown in the figure). The image generating unit 104 performs a weighing appropriate for the frequency band so as to level the average pixel level of the image data when generating image data.

Fig. 16 shows how the image data are generated using the basic wave ω1, the second harmonics ω2 and the third harmonics ω3 to the respective RF signals at the three steering angle directions (θ1, θ2 and θ3), and nine image data in total are stored.

These nine image data generated in this way are synthesized by the image generating unit 106. Fig. 16 shows two synthesized methods.

Synthesis example 1 is the case where frequencies are compound first (images synthesized using different frequency bands in the same steering angle direction are synthesized), after that, these synthesized images are further synthesized to make the final synthesized image.

Synthesis example 2 is the case where the above-mentioned synthesis sequence is inversed, that is, images generated using the same frequency band at different steering angles are synthesized first, and then these synthesized images are further synthesized (frequencies are compound) to make the final synthesized image.

Those two synthesis examples are mathematically equal to each other, and the final result should not be affected by the processing sequence, however, some influence appears on the final image because some variable components such as column missing or round-off errors are inevitably included in the real processing and the influence of variable components varies depending on the synthesis sequence. As the sequence of the synthesis processing differs depending on which method is selected as a synthesis method, it is desirable to equip a memory on which the relation between the processing method and the processing sequence is previously registered or let a user specify the synthesis method or the processing sequence as the need arises.

The processing amount increases like mentioned above when synthesizing an image when off-line, but it is possible to improve the noise elimination effect because the number of data used for generating synthesized images also increases.

However, when the number of image data used for synthesis increases, the synthesized image data blurs accordingly. In this case, it is advisable to lessen the blur on the synthesized image data by using a synthesis method such as the HPF (High Pass Filter), an edge emphasis filter or another synthesis method whose effect is equivalent.

It is needless to say that functional block diagrams shown in Fig. 4, 9 and 13 that are referred to in embodiments respectively and the flow chart as Fig. 11 can be realized as a program in the DSP or the CPU in the apparatus body 12 shown in Fig. 3. This program is distributable via a recording medium such as a CD or an electric communication circuit, and it can be independently traded as a package software or a software for download.

Although the present invention has been fully described by way of examples with reference to the accompanying drawings, it is to be noted that various changes and modifications will be apparent to those skilled in the art. Therefore, unless otherwise such changes and modifications depart from the scope of the present invention, they should be construed as being included there in.

## Claims

1. An image processing apparatus for sending ultrasound from an ultrasound probe and generating an image based on reflection signals generated from echoes from a check object, the image processing apparatus comprising:

   a specifying unit operable to specify at least two frequency bands in reflection signals;
   a generating unit operable to generate images for each of the specified frequency bands based on each of signals having the specified frequency bands in the reflection signals; and
   a synthesizing unit operable to synthesize the generated images for the respective frequency bands.

**2.** The image processing apparatus according to Claim 1,

wherein the synthesizing unit synthesizes the "i" th pixel value f_g(i) of a synthesized image according to a pixel value calculation shown in formula 1, M being the number of the images for the specified frequency bands generated by the generating unit, N being the number of pixels of the respective images, f_m(i) being an "i" th pixel value of a "m" th image in the generated images, and S being a value within a range of the possible f_m(i).

(formula 1)

$$f\_g(i)=S-M/((1/(S-f\_0(i)))+(1/(S-f\_1(i)))+...+(1/(S-f\_{(M-1)}(i))))$$

**3.** The image processing apparatus according to Claim 1, further comprising an area discriminating unit operable to discriminate a first area including edges from the other areas based on the images generated by the generating unit,

wherein the synthesizing unit performs a first synthesis calculation as to pixels in a first area of the images generated by the generating unit and a calculation different from the first synthesis calculation as to pixels in the other areas.

**4.** The image processing apparatus according to Claim 3,

wherein the area discriminating unit includes:

a threshold judging unit operable to make a threshold judgment pixel by pixel as to the respective images generated by the generating unit; and
an area data generating unit operable to generate area data showing distributions of the first area and the other areas by taking logical OR of threshold judgment results on the respective images.

**5.** The image processing apparatus according to Claim 4,

wherein the threshold judgment unit makes the threshold judgment regarding a limit of a possible pixel value of pixels around an edge as threshold.

**6.** The image processing apparatus according to Claim 3,

wherein the synthesizing unit includes:

at least two calculation units in a first to a fourth calculation units; and
a selecting unit operable to select at least two calculation units for the respective pixels, one being a calculation unit for performing the first synthesis calculation on the pixels in the first area and another being a calculation unit for performing another synthesis calculation on the pixels in the other areas, according to area data so as to have the selected calculation units calculate pixel values;
the first calculation unit synthesizes pixels using a maximum value as to the corresponding pixels of a plurality of images generated by the generating unit,
the second calculation unit synthesizes pixels performing arithmetic averaging as to the corresponding pixels of a plurality of images,
the third calculation unit synthesizes pixels performing harmonic averaging as to the corresponding pixels of a plurality of images, and
the fourth calculation unit synthesizes the "i" th pixel value f_g(i) of a synthesized image according to a pixel value calculation shown in formula 2, M being the number of the plurality of images, N being the number of pixels of the respective images, f_m(i) being an "i" th pixel value of a "m" th image in the generated images, and S being a value within a range of a possible f_m(i).

(formula 2)

$$f\_g(i)=S-M/((1/(S-f\_0(i)))+(1/(S-f\_1(i)))+...+(1/(S-f\_{(M-1)}(i))))$$

**7.** The image processing apparatus according to Claim 1,

wherein the ultrasound probe sends ultrasound for a plurality of steering angles,
the specifying unit specifies the different frequency bands as to at least two steering angles in the plurality of steering angles,
the generating unit generates images for each of the specified frequency bands based on each of the signals having the specified frequency band in reflection signals for the respective steering angles, and

the synthesizing unit synthesizes images for the respective generated steering angles.

8. The image processing apparatus according to Claim 7,

wherein the specifying unit specifies a different frequency band for a predetermined steering angle in the plurality of steering angles.

9. The image processing apparatus according to Claim 7,

wherein the specifying unit equips an association table for storing a plurality of sets including at least a first and a second sets, each of which is made of a steering angle group and a frequency band group associating with the plurality of steering angles,

the image processing apparatus controls the steering angle of the ultrasound probe based on the steering angle group in one of the plurality of sets stored in the association table,

the specifying unit specifies the frequency band to the respective steering angles based on the frequency band group associating with the steering angle group in the current set, and

in the association table, a difference between frequency bands in the first frequency band group is designed to be bigger than a difference between frequency bands in the second frequency band group when a difference between steering angles in the first steering angle group is smaller than a difference between steering angles in the second steering angle group.

10. The image processing apparatus according to Claim 7,

wherein the synthesizing unit synthesizes the "i" th pixel value $f\_g(i)$ of a synthesized image according to a pixel value calculation shown in formula 3, M being the number of the images for the respective steering angles generated by the generating unit, N being the number of pixels of the respective images, $f\_m(i)$ being an "i" th pixel value of a "m" th image in the generated images, and S being a value within a range of a possible $f\_m(i)$.


(formula 3)


$$f\_g(i)=S-M/((1/(S-f\_0(i)))+(1/(S-f\_1(i)))+...+(1/(S-f\_(M-1)(i))))$$


11. The image processing apparatus according to Claim 7, further comprising an area discriminating unit operable to discriminate a first area including edges from the other areas based on the images for the respective steering angles generated by the generating unit,

wherein the synthesizing unit performs a first synthesis calculation as to pixels in a first area of the images for the respective steering angles generated by the generating unit and a calculation different from the first synthesis calculation as to pixels in the other areas.

12. The image processing apparatus according to Claim 11,

wherein the area discriminating unit includes:

a threshold judging unit operable to make a threshold judgment pixel by pixel as to the respective images generated by the generating unit; and

an area data generating unit operable to generate area data showing distributions of the first area and the other areas by performing logical OR of threshold judgment results on the respective images.

13. The image processing apparatus according to Claim 12,

wherein the threshold discriminating unit makes a threshold judgment regarding the limit of the possible pixel value of a pixel around an edge as threshold.

14. The image processing apparatus according to Claim 11,

wherein the synthesizing unit includes:

at least two calculation units in a first to a fourth calculation units; and

a selecting unit operable to select at least two calculation units for the respective pixels, one being a calculation unit for performing a synthesis calculation on the pixels in the first area or another being a calculation unit for performing another synthesis calculation on the pixels in the other areas, according to the area data so as to have the calculation unit calculate pixel values;

the first calculation unit synthesizes pixels using a maximum value as to pixels of a plurality of images generated by the generating unit,

the second calculation unit synthesizes pixels performing arithmetic averaging as to pixels of a plurality of images,

the third calculation unit synthesizes pixels performing harmonic averaging as to pixels of a plurality of images, and

the fourth calculation unit synthesizes the "i" th pixel value f_g(i) of a synthesized image according to a pixel value calculation shown in formula 4, M being the number of the images generated for the respective steering angles, N being the number of pixels of the respective images, f_m(i) being an "i" th pixel value of a "m" th image in the generated images, and S being a value within a range of a possible f_m(i).

(formula 4)

$$f\_g(i)=S-M/((1/(S-f\_0(i)))+(1/(S-f\_1(i)))+...+(1/(S-f\_(M-1)(i))))$$

15. The image processing apparatus according to Claim 14, further comprising a storing unit operable to store the reflection signals received by the ultrasound probe,

wherein the generating unit and synthesizing unit are formed so that they can select either way of generating and synthesizing images in real time based on the reflection signals received by the ultrasound probe or when off-line based on the reflection signals stored in the storage unit.

16. The image processing apparatus according to Claim 15,

wherein the specifying unit specifies a plurality of frequency bands for the respective steering angles when off-line,

the generating unit generates images for the plurality of frequency bands for the respective steering angles when off-line,

the synthesizing unit generates a first and a second synthesized images,

the first synthesized image is obtained by further synthesizing synthesized images for the respective steering angles after synthesizing images derived from different frequency bands at the same steering angle, and

the second synthesized image is obtained by further synthesizing synthesized images for the respective frequency bands after synthesizing images derived from the same frequency band obtained in different steering angles.

17. An image processing method for sending ultrasound from an ultrasound probe and generating an image based on reflection signals from a check object, comprising:

a specifying step of specifying at least two frequency bands in reflection signals;

a generating step of generating images for each of the specified frequency bands based on each of the signals having the specified frequency bands in the reflection signals; and

a synthesizing step of synthesizing the generated images for the respective generated frequency bands.

18. The image processing method according to Claim 17,

wherein the ultrasound probe sends ultrasound at a plurality of steering angles,

in the specifying step, different frequency bands are specified respectively for at least two steering angles in the plurality of steering angles,

in the generating step, images are generated for the respective frequency bands based on the signal having the specified frequency band in reflection signals respectively for the steering angles, and

in the synthesizing step, the images generated for the respective steering angles are synthesized.

19. A program capable of being executed by a computer in an image processing apparatus for sending ultrasound from an ultrasound probe and generating images based on reflection signals from a check object, and causing the computer to execute the following steps:

a specifying step of specifying at least two frequency bands in reflection signals;

a generating step of generating images for each of the frequency bands based on each of the signals having the specified frequency bands in reflection signals; and

a synthesizing step of synthesizing the generated images for the respective frequency bands.

**20.** The program according to Claim 19,
wherein the ultrasound probe sends ultrasound at a plurality of steering angles,
in the specifying step, different frequency bands are specified respectively for at least two steering angles in the plurality of steering angles,
in the generating step, images are generated for the respective frequency bands based on the signal having the specified frequency band in reflection signals respectively for the steering angles, and
in the synthesizing step, the images generated for the respective steering angles are synthesized.

Fig. 1A

13

Fig. 1B

b1

Fig. 1C

c1 · c4

c2 · c5

c3 · c6

c7
c8
c9

c10

synthesis
c11

## Fig. 2A

13

check
object

$\theta$

☒ Scanning area under steering angle of $\theta$ or $\theta'$

☐ Scanning area under steering angle of 0

▨ Scanning area under steering angle of $-\theta$ or $-\theta'$

## Fig. 2B

13

$\theta'$

check
object

EP 1 411 370 A1

Fig. 3

## Fig. 4

```
        ┌──────────────────┐ 101
        │ Array ultrasound │
        │ probe            │
        └────────┬─────────┘
                 │
                 ▼
   ┌──────────────────────┐ 102          ┌──────────────────────┐ 103
   │ Sending and receiving│◄─────────────│ Steering angle       │
   │ control unit         │              │ indicating unit      │
   └──────────┬───────────┘              └──────────┬───────────┘
              │                                     │
              ▼                                     ▼
   ┌──────────────────────┐ 104          ┌──────────────────────┐ 105
   │ Image generating unit│◄─────────────│ Frequency band       │
   │                      │              │ selecting unit       │
   └──────────┬───────────┘              └──────────────────────┘
              │
              ▼
   ┌──────────────────────┐ 106
   │ Image synthesizing   │
   │ unit                 │              ┌──────────────────────┐ 107
   └──────────┬───────────┘◄────────────►│ Image storing unit   │
              │                          └──────────────────────┘
              ▼
   ┌──────────────────────┐ 108
   │ Frame memory         │
   └──────────┬───────────┘
              │
              ▼
   ┌──────────────────────┐ 109
   │ Image display unit   │
   └──────────────────────┘
```

## Fig. 5

T1

| no. | $\theta 1$ | $\theta 2$ | $\theta 3$ |
|-----|-----|-----|-----|
| 1 | -5° | 0° | 5° |
| 2 | -10° | 0° | 10° |
| 3 | -15° | 0° | 15° |
| ⋮ | ⋮ | ⋮ | ⋮ |

Pointer P

## Fig. 6A

T2

| no. | $\theta 1$ | $\theta 2$ | $\theta 3$ |
|-----|-----|-----|-----|
| 1 | $\omega 1$ | $\omega 2$ | $\omega 3$ |
| 2 | $\omega 3$ | $\omega 2$ | $\omega 1$ |
| 3 | $\omega 2$ | $\omega 1$ | $\omega 3$ |
| 4 | $\omega 1$ | $\omega 2$ | $\omega 1$ |
| 5 | $\omega 2$ | $\omega 1$ | $\omega 2$ |
| ⋮ | ⋮ | ⋮ | ⋮ |

← Pointer Q

## Fig. 6B

T3

| P | Q |
|---|---|
| 1 | 1 |
| 2 | 2 |
| 3 | 4 |
| | |
| | |

## Fig. 7

# Fig. 8

## Fig. 9

Fig. 10A

Array ultrasound probe ~13

Subject A ⊙

Subject B ⊙

Subject C ⊙

Echo A     Echo B     Echo C     Synthesized echo

d2 d1 depth

n1

When generating images using basic wave

Fig. 10B

Array ultrasound probe ~13

Subject A ⊙

Subject B ⊙

Subject C ⊙

Echo A     Echo B     Echo C     Synthesized echo

d2 d1 depth

n2

When generating images using second harmonics

EP 1 411 370 A1

# Fig. 11

Start

Loop 1:i =1~3 — Step 80

Specify steering angle of $\theta i$ — Step 81

Sending and receiving under specified steering angle — Step 82

Select frequency band appropriate for $\theta i$ — Step 83

Generate image from received wave of selected frequency band — Step 84

Loop 1:end — Step 85

Synthesize generated images — Step 86

End

Fig. 12

Array ultrasound probe — 101

Sending and receiving control unit — 102

Steering angle indicating unit — 103

Frequency band selecting unit — 105

Image generating unit — 104

Threshold setting unit — 201

Threshold mask generating unit — 202

Image synthesizing unit — 200

Maximum value detecting unit — 203

Arithmetic average calculation unit — 204

Harmonic average calculation unit — 205

Pixel value calculation unit — 206

Synthesizing method selecting unit — 207

Image storing unit — 107

Frame memory — 108

Image display unit — 109

Fig. 13

□ Mask value: 1
■ Mask value: 0

f_1(i) f_2(i) f_3(i)

T_f1(i) T_f2(i) T_f3(i)

□ Mask value: 1
■ Mask value: 0

T_g(i)

## Fig. 14

```
            ( Start )
                |
                v
   [ Loop 1:each pixel ]        Step 101
                |
                v
   [ Determine area to which    Step 102
     current pixel belong ]
                |
                v         Step 103
          < Area 0/1 ? > -----------------+
                |                          |
                v  Step 104               v  Step 105
   [ Select calculation for    [ Select calculation for
     area 0 ]                    area 1 ]
                |                          |
                v <------------------------+
   [ Synthesize pixels by       Step 106
     selected calculation ]
                |
                v
   [ Loop 1:End ]               Step 107
                |
                v
            ( End )
```

Fig. 15

Array ultrasound probe — 101

Sending and receiving control unit — 102

Steering angle indicating unit — 103

RF signal storing unit — 301

Image generating unit — 104

Frequency band selecting unit — 105

Threshold setting unit — 201

Threshold mask generating unit — 202

Image synthesizing unit

Maximum value detecting unit — 203

Arithmetic average calculation unit — 204

Synthesizing method selecting unit — 207

— 200

Harmonic average calculation unit — 205

Pixel value calculation unit — 206

Image storing unit — 107

Frame memory — 108

Image display unit — 109

EP 1 411 370 A1

Fig. 16

Sending and receiving control unit ~102

301

RF signal storaging unit

104

Image generating unit

Image data memory

Basic wave
Second harmonics
Third harmonics

θ1    θ2    θ3

Synthesis example 2

Synthesis example 1

Basic wave
Second harmonics
Third harmonics

b1
b2
b3
b4

S2

S1

θ1    θ2    θ3

a1
a2
a3
a4

Selection    S3

To image storing unit, frame memory

Image synthesizing unit    200

EP 1 411 370 A1

## EUROPEAN SEARCH REPORT

**European Patent Office**

**Application Number**

EP 03 02 3280

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 879 303 A (BURNS PETER N ET AL) 9 March 1999 (1999-03-09) * abstract * * figure 14 * | 1,17,19 | G01S15/89 G01S7/52 A61B8/14 |
| A | | 2 | |
| X | US 5 873 829 A (MINE YOSHITAKA ET AL) 23 February 1999 (1999-02-23) * abstract * * figures 6,7 * | 1,17,19 | |
| A | | 2 | |
| X | DE 34 13 074 A (SIEMENS AG) 17 October 1985 (1985-10-17) | 1,7-9, 17-20 | |
| Y | * abstract * * page 10, line 15 – page 18, line 23 * * figures 1,3,4,6 * | 11 | |
| A | | 2 | |
| X | US 6 048 316 A (ZHAO DANHUA ET AL) 11 April 2000 (2000-04-11) | 1,3,17, 19 | |
| Y | * abstract * * column 2, line 16 – column 4, line 27 * * figures 1-6 * | 11 | TECHNICAL FIELDS SEARCHED (Int.Cl.7) G01S A61B |
| A | | 2,4-6, 10,12-16 | |
| X | US 6 224 553 B1 (NEVO EREZ) 1 May 2001 (2001-05-01) * abstract * * figure 1 * | 1,17,19 | |
| A | | 2 | |

-/--

The present search report has been drawn up for all claims

| Place of search | Date of completion of the search | Examiner |
|---|---|---|
| MUNICH | 16 February 2004 | Willig, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

## EUROPEAN SEARCH REPORT

Application Number

EP 03 02 3280

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 2001/032512 A1 (OHTSUKI SHIGEO ET AL) 25 October 2001 (2001-10-25) <br> * column 6, line 28 - column 7, line 27 * <br> * figure 3 * | 1,2,17, 19 | |
| A | US 5 664 572 A (KISHIMOTO SHINJI) 9 September 1997 (1997-09-09) <br> * abstract * <br> * figures 1,2 * | 1,2,17, 19 | |
| A | US 6 123 670 A (MO LARRY Y L) 26 September 2000 (2000-09-26) <br> * abstract * <br> * figure 4 * | 3 | |
| A | US 6 443 894 B1 (SCHLESINGER RANDALL ET AL) 3 September 2002 (2002-09-03) <br> * abstract * | 3 | |
| A | US 5 908 390 A (MATSUSHIMA TETSUYA) 1 June 1999 (1999-06-01) <br> * column 18, line 1-34 * <br> * column 20, line 26-34,48-56 * <br> * figures 5-8 * | 7-9,18, 20 | **TECHNICAL FIELDS SEARCHED (Int.Cl.7)** |
| A | ENTREKIN R ET AL: "Real time spatial compound imaging in breast ultrasound: technology and early clinical experience" MEDICAMUNDI, PHILIPS MEDICAL SYSTEMS, SHELTON, CT,, US, vol. 43, no. 3, September 1999 (1999-09), pages 35-43, XP002181821 ISSN: 0025-7664 * the whole document * | 7-9,18, 20 | |

-/--

The present search report has been drawn up for all claims

| Place of search | Date of completion of the search | Examiner |
|---|---|---|
| MUNICH | 16 February 2004 | Willig, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 411 370 A1**

**European Patent Office**

**EUROPEAN SEARCH REPORT**

Application Number

EP 03 02 3280

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | JESPERSEN S K ET AL: "ULTRASOUND SPATIAL COMPOUND SCANNER FOR IMPROVED VISUALIZATION IN VASCULAR IMAGING" 1998 IEEE ULTRASONICS SYMPOSIUM PROCEEDINGS. SENDAI, MIYAGI, JP, OCT. 5 – 8, 1998, IEEE ULTRASONICS SYMPOSIUM PROCEEDINGS, NEW YORK, NY: IEEE, US, vol. 2, 5 October 1998 (1998-10-05), pages 1623-1626, XP000871850 ISBN: 0-7803-4096-5 * the whole document * | 7-9,18, 20 | |
|  |  |  | **TECHNICAL FIELDS SEARCHED (Int.Cl.7)** |

The present search report has been drawn up for all claims

| Place of search | Date of completion of the search | Examiner |
|---|---|---|
| MUNICH | 16 February 2004 | Willig, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

34

| ))) | European Patent | Application Number |
|---|---|---|
| | Office | EP 03 02 3280 |

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**European Patent Office**

**LACK OF UNITY OF INVENTION
SHEET B**

**Application Number**

EP 03 02 3280

The Search Division considers that the present European patent application does not comply with the requirements of unity of invention and relates to several inventions or groups of inventions, namely:

1. Claims: 1,2,17,19

    Image processing apparatus, method and program using a pixel value calculation formula for synthesizing the generated images.

2. Claims: 1,3-6

    Image processing apparatus with an area discriminating unit.

3. Claims: 1,7-20

    Image processing apparatus, method and program using different steering angles for different frequency bands.

ANNEX TO THE EUROPEAN SEARCH REPORT
ON EUROPEAN PATENT APPLICATION NO.

EP 03 02 3280

This annex lists the patent family membersrelating to the patent documents cited in the above–mentioned European search report.
The members are as contained in the European Patent Office EDP file on
The European Patent Office is in no way liable for these particulars which are merely given for the purpose of information.

16-02-2004

| Patent document cited in search report | | Publication date | Patent family member(s) | | Publication date |
|---|---|---|---|---|---|
| US 5879303 | A | 09-03-1999 | US | 5833613 A | 10-11-1998 |
| | | | US | 5908389 A | 01-06-1999 |
| | | | US | 6283919 B1 | 04-09-2001 |
| | | | US | 6251074 B1 | 26-06-2001 |
| | | | US | 6458083 B1 | 01-10-2002 |
| | | | US | 2002040189 A1 | 04-04-2002 |
| | | | US | 2002055681 A1 | 09-05-2002 |
| | | | US | 6315729 B1 | 13-11-2001 |
| | | | EP | 0851241 A2 | 01-07-1998 |
| US 5873829 | A | 23-02-1999 | JP | 9201359 A | 05-08-1997 |
| DE 3413074 | A | 17-10-1985 | DE | 3413074 A1 | 17-10-1985 |
| US 6048316 | A | 11-04-2000 | NONE | | |
| US 6224553 | B1 | 01-05-2001 | AU | 6673498 A | 29-09-1998 |
| | | | WO | 9840014 A1 | 17-09-1998 |
| US 2001032512 | A1 | 25-10-2001 | JP | 2001166040 A | 22-06-2001 |
| US 5664572 | A | 09-09-1997 | JP | 9094248 A | 08-04-1997 |
| US 6123670 | A | 26-09-2000 | DE | 19960078 A1 | 21-06-2000 |
| | | | JP | 2000232978 A | 29-08-2000 |
| US 6443894 | B1 | 03-09-2002 | NONE | | |
| US 5908390 | A | 01-06-1999 | NONE | | |

EPO FORM P0459

For more details about this annex : see Official Journal of the European Patent Office, No. 12/82